(19) Europäisches Patentamt
European Patent Office
Office européen des brevets

(11) Veröffentlichungsnummer : **0 107 139**
**B1**

(12) # EUROPÄISCHE PATENTSCHRIFT

(45) Veröffentlichungstag der Patentschrift : **29.07.87**

(51) Int. Cl.[4] : **C 07 D251/50**, D 21 H 3/02

(21) Anmeldenummer : **83110118.3**

(22) Anmeldetag : **11.10.83**

(54) **Monofluortriazinyl-Verbindungen, Verfahren zu ihrer Herstellung und ihre Verwendung als Leimungsmittel.**

(30) Priorität : **23.10.82 DE 3239366**

(43) Veröffentlichungstag der Anmeldung : **02.05.84 Patentblatt 84/18**

(45) Bekanntmachung des Hinweises auf die Patenterteilung : **29.07.87 Patentblatt 87/31**

(84) Benannte Vertragsstaaten : **CH DE FR GB IT LI SE**

(56) Entgegenhaltungen :
**CH-A- 599 179**
**GB-A- 1 157 787**
**US-A- 3 397 204**

(73) Patentinhaber : **BAYER AG**
**Konzernverwaltung RP Patentabteilung**
**D-5090 Leverkusen 1 Bayerwerk (DE)**

(72) Erfinder : **Sackmann, Günter, Dr.**
**Friedenberger Strasse 11**
**D-5090 Leverkusen 3 (DE)**
Erfinder : **Jäger, Horst, Dr.**
**Carl-Rumpff-Strasse 37**
**D-5090 Leverkusen (DE)**
Erfinder : **Beck, Ulrich, Dr.**
**Weiherstrasse 17**
**D-5303 Bornheim 3 (DE)**
Erfinder : **Bäumgen, Heinz**
**Buchenweg 10**
**D-5090 Leverkusen (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Jouve, 18, rue St-Denis, 75001 Paris, France

**Beschreibung**

Die Leimung von Papier auf einer Papiermaschine wird in der Regel mit synthetischen oder natürlichen Leimungsmitteln durchgeführt. Die Leimung kann dabei nach zwei unterschiedlichen Wirkungsmechanismen eintreten, je nachdem welcher Typ von Leimungsmittel zum Einsatz gelangt. Werden nicht-reaktive Leimungsmittel, wie z. B. Harzleim, Paraffin oder bestimmte synthetische Hochpolymere, zusammen mit kationischen Fixiermitteln, wie z. B. Aluminiumsulfat oder kationisch geladenen Polymeren verwendet, dann wird die Leimung durch eine ionische Fixierung der Produkte auf den Cellulosefasern bewirkt. Beim Einsatz von Reaktivleimungsmitteln wird die Leimung durch die Entstehung kovalenter Bindungen zwischen Leimungsmittel und Zellstoff hervorgerufen. Als Reaktivleimungsmittel bezeichnet man hydrophobe Substanzen mit solchen funktionellen Gruppen, die zur chemischen Reaktion mit dem OH-Gruppen der Cellulose befähight sind. Als Beispiele für Reaktivleimungsmittel seien genannt :

Stearoyldiketen, Tetrapropenylbernsteinsäureanhydrid, Stearylisocyanat, Dehydroabietylisocyanat, Epoxide und Derivate von Cyanurchlorid.

Die Reaktivleimungsmittel haben gegenüber den nicht-reaktiven Leimungsmitteln den Vorteil, daß sie eine größere Effektivität besitzen, da sie fest an die Fasern gebunden sind. Dadurch sind die zur Erzielung einer guten Leimungswirkung benötigten Leimungsmittelmengen wesentlich niedriger als bei der Verwendung nicht-reaktiver Leimungsmittel.

Als Reaktivleimungsmittel haben nich am Markt vor allem solche Handelsprodukte durchgesetzt, die Stearoyldiketen als Wirksubstanz enthalten.

Diese Produkte zeigen in Form von wäßrigen Suspensionen oder Lösungen in organischen Lösungsmitteln, besonders auf im neutralen oder schwach alkalischen Medium hergestellten Papieren, gute bis sehr gute Leimungseigenschaften. Unter neutral oder schwach alkalisch gefahrenen Papieren versteht man gewöhnlich solche Papiere, die mit $CaCO_3$ als Füllstoff und ohne den Zusatz von Aluminiumsulfat gefahren werden. Nachteilig bei Leimungsmitteln auf Stearoyldiketen-Basis ist jedoch, daß diese auf Papieren, die nicht mit Kreide, sondern mit Kaolin als Füllstoff hergestellt werden, nur eine sehr geringe oder aber überhaupt keine Leimungswirkung zeigen.

Es ist deshalb von Interesse, solche Leimungsmittel herzustellen, die universell einsetzbar sind, d. h. sowohl auf neutralen als auch auf sauren Papieren, sowie auf Papieren, die Kreide oder Kaolin als Füllstoff enthalten.

Aufgabe der Erfindung war deshalb die Bereitstellung eines Reaktivleimungsmittels für Papier, das sowohl auf sauer als auch auf neutral bis alkalisch gefahrenen Papieren einsetzbar ist und dessen Wirksamkeit unabhängig vom Papierfüllstoff und der Anwesenheit von Aluminiumsulfat im Papier ist.

Es wurde nur gefunden, daß die neuen Monofluortriazinyl-Verbindungen der Formel

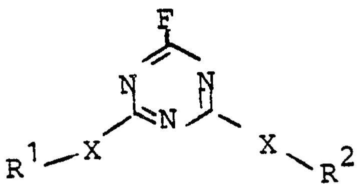

(1)

worin

$R^1$ und $R^2$ gleich oder verschieden sein können und für einen Kohlenwasserstoffrest mit 12 bis 24 C-Atomen stehen,

X für ein Sauerstoffatom, ein Schwefelatom oder die Gruppe $NR^3$ steht, worin

$R^3$ Wasserstoff oder einen Kohlenwasserstoffrest mit 1 bis 24 C-Atomen bedeutet, sehr gut als universell einsetzbare Wirksubstanzen für Leimungsmittel geeignet sind.

Für Formel (1) gilt :

Der Kohlenwasserstoffrest $R^1$ und $R^2$ steht vorzugsweise für lineares oder verzweigtes Alkyl ($C_{12}$-$C_{24}$) oder für Cycloalkyl ($C_{12}$-$C_{24}$) oder für aromatische Reste mit anellierten hydroaromatischen Systemen mit 12 bis 24 C-Atomen. Der Kohlenwasserstoffrest $R^3$ steht vorzugsweise für die vorgenannten Substituenten und für Methyl.

Die Erfindung betrifft bevorzugt Verbindungen der Formeln (2), (3) und (4) :

(Siehe Formeln Seite 3 f.)

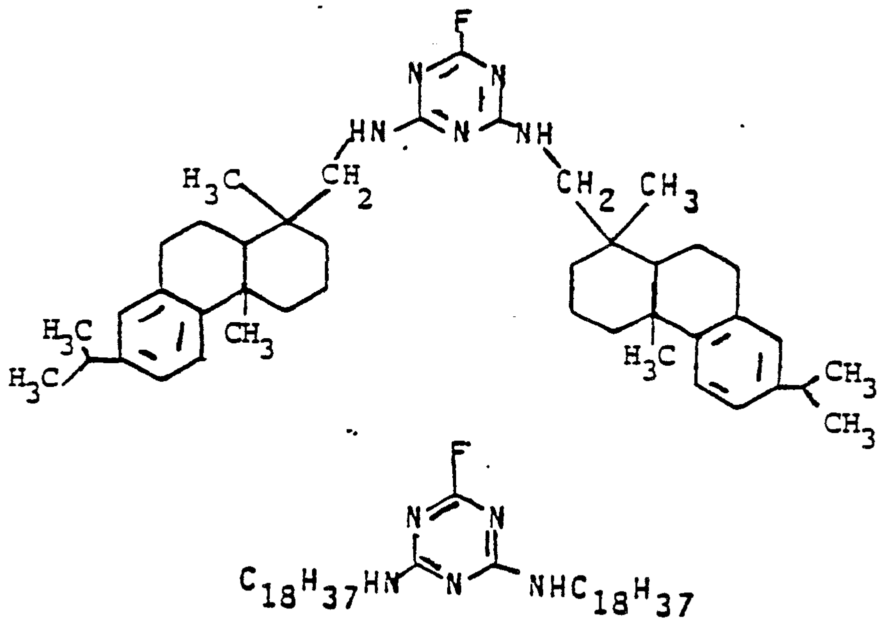

(2)

(3)

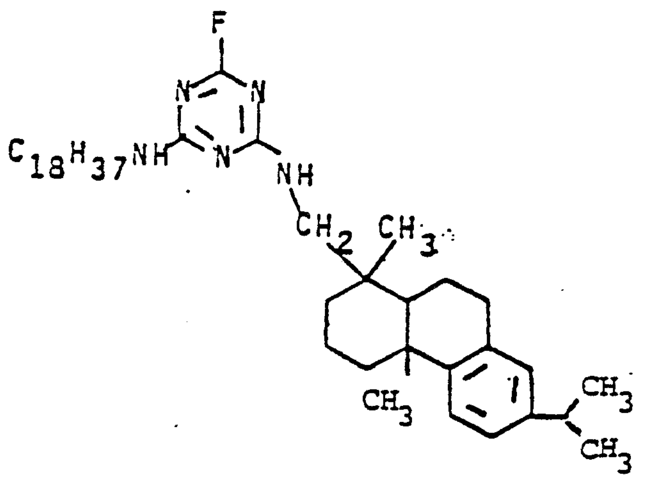

(4)

Die Erfindung betrifft weiterhin Papierleimungsmittel enthaltend als Wirksubstanz eine Verbindung der Formeln (1), (2), (3) oder (4).

Bevorzugte Leimungsmittel enthalten als Zusatz ein kationisches Fixiermittel.

Gegenstand der Erfindung ist weiterhin ein Verfahren zur Leimung von Papier, dadurch gekennzeichnet, daß Verbindungen der allgemeinen Formel

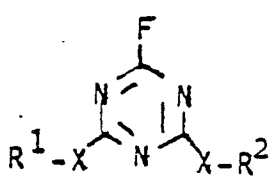

(1)

wobei $R^1$, $R^2$ und X die oben genannte Bedeutung haben,
entweder alleine oder in Kombination mit kationischen Fixiermitteln bei der Papierherstellung eingesetzt werden.

Die Verbindungen der Formel (1) werden hergestellt, indem man Trifluortriazin (TFT) mit Monoaminen, Monoalkoholen oder Mercaptanen der allgemeinen Formeln

$$R^1\text{—}XH \quad \text{und/oder} \quad R^2\text{—}XH$$

umsetzt, wobei

$R^1$, $R^2$ und X die oben angegebene Bedeutung besitzen.

Als kationische Fixiermittel in Kombination mit den Monofluortriazinderivaten der Formel (1) kommen vorzugsweise Aluminiumsalze (insbesondere Aluminiumsulfat) und kationisch geladene Polymere in Frage. Als derartige Polymere seien aufgeführt : Copolymere aus Acrylamid und Dimethylaminoethylmethacrylat, kationische Polyamidamine, harnstoffgruppenhaltige Polyamine, Polyimine und kationische Stärke.

Als Beispiele für die zum Einsatz gelangenden Monoamine bzw. Monoalkohole der Formeln $R^1$—XH und $R^2$—XH seien genannt :

Dodecylamin, Hexadecylamin, Stearylamin, Methyloctadecylamin, Dodecanol, Hexadecanol, Stearylalkohol oder Gemische davon.

Neben linearen oder verzweigten aliphatischen Monoaminen oder Monoalkoholen können aber auch mehrkernige, cycloaliphatische Monoamine oder Monoalkohole eingesetzt werden. Als Vertreter dieser Gruppe seien z. B. Abietylamin und Dehydroabietylamin sowie Abietylalkohol und Dehydroabietylalkohol genannt.

Die Reaktivität der Fluoridgruppe von mit o. g. Monoaminen oder Monoalkoholen substituierten Trifluortriazinen gegenüber Cellulose ist so hoch, daß im Gegensatz zu Cyanurchlorid, wo zwei Cl-Gruppen nötig sind, bereits ein Fluorsubstituent genügt, um eine gute chemische Fixierung des Leimungsmittels auf der Papierfaser und damit eine ausgezeichnete Leimungswirkung zu erreichen.

In einer bevorzugten Ausführungsform der Erfindung kommen folgende Verbindungen (2), (3) und (4) als Leimungsmittel bei der Papierherstellung zum Einsatz :

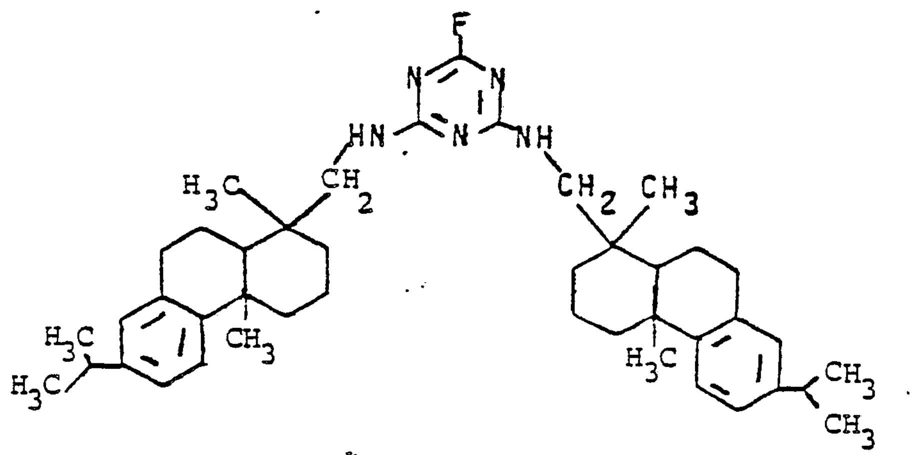

(2)

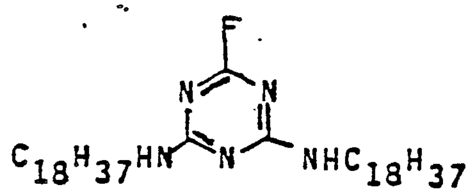

(3)

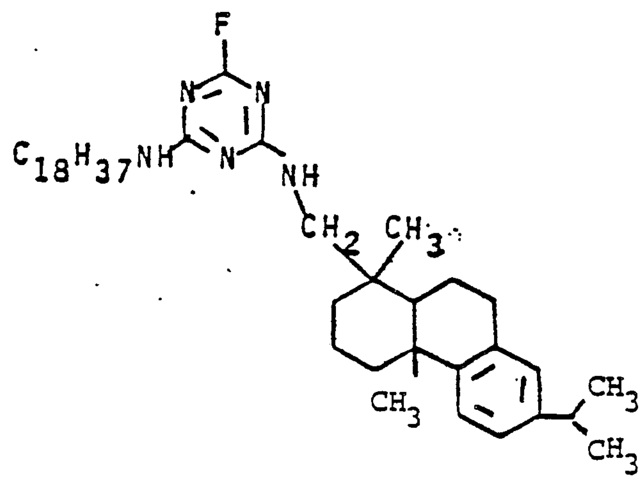

(4)

Bei dem erfindungsgemäßen Papierleimungsverfahren kann es sich sowohl um eine Oberflächenleimung als auch um eine Masseleimung handeln. Dazu können die genannten hydrophoben Monofluortriazin-Derivate der Formel (1) in Form von Lösungen, z. B. in organischen Lösungsmitteln wie Toluol oder Xylol oder in Form von wäßrigen Zubereitungen, etwa in Form von Suspensionen oder Emulsionen,

Verwendung finden. Als Suspendierhilfsmittel sind z. B. Polyvinylalkohol oder wasserlösliche Cellulosederivate, insbesondere kationische Stärke gut geeignet, gegebenenfalls in Kombination mi vorzugsweise nicht-ionischen Emulgatoren. Zur Beschleunigung der Reaktion zwischen den beschriebenen Verbindungen und der Cellulosefaser können den Leimungsmittelpräparationen auf wäßriger Basis auch schwach alkalische Substanzen wie $NaHCO_3$ oder $Na_2CO_3$ zugesetzt werden.

Beispiel 1

45 g 92 %iges Dehydroabietylamin (0,145 mol) werden in 500 ml getrocknetem Toluol gelöst. Man gibt dann 14,8 g Triethylamin (0,145 mol) hinzu und tropft bei 0-5° 9,75 g Trifluortriazin (0,072 mol) ein. Anschließend erwärmt man auf 20-25 °C und rührt zwei Stunden bei dieser Temperatur. Zur Entfernung des Triethylaminhydrofluorids schüttelt man zweimal mit je 100 ml Wasser aus. Man erhält 530 ml einer 9 %igen toluolischen Lösung von 2-Fluor-4,6-di-dehydroabietylaminotriazin.

Beispiel 2

Einsatz des gemäß Beispiel 1 hergestellten Produktes als Leimungsmittel : Um die Leimungseigenschaften des nach Beispiel 1 hergestellten Monofluortriazinderivats möglichst störungsfrei zu ermitteln, d. h. um alle durch die Formulierung bedingten Einflüsse auszuschalten, wird der unten beschriebene Test mit verschiedenen Papiersorten durchgeführt. Die dabei eingesetzten Papiere hatten folgende Zusammensetzung :

A) neutral : 50 Tle. Nadelholzsulfat
50 Tle. Birkensulfat
10 Tle. Kreide
B) neutral : 50 Tle. Nadelholzsulfat
50 Tle. Birkensulfat
10 Tle. Kaolin
C) sauer : 50 Tle. Nadelholzsulfat
50 Tle. Birkensulfat
12 Tle. Kaolin
1,5 Tle. Alaun

Diese Papiere wurden auf einem Foulard-Gerät mit den zu prüfenden Leimungsmitteln, 0,1 bzw. 0,2 %ig gelöst in Toluol, ausgerüstet und abgequetscht. Danach wurden sie den im folgenden beschriebenen Nachbehandlungen I-IV unterzogen

| | Art der Nachbehandlung | zu untersuchender Effekt |
|---|---|---|
| I | 5 Min. bei 150 °C | Wirkung des ausreagierten Leimungsmittels |
| II | 1 Min. bei 90 °C | Start der Reaktion des reinen Wirkstoffs bei praxisüblichen Temperaturen 120 °C |
| III | 5 Min. bei 90 °C zwischen 2 nassen Filterpapieren | Einfluß der Feuchtigkeit |
| IV | Luftgetrocknet bei 20 °C | eventuelle Leimung ohne chemische Reaktion |

Die geleimten und nach I-IV nachbehandelten Papiere werden in Streifen von 3 cm Breite und 9 cm Länge geschnitten und auf blaue Prüftinte gelegt. Nach 1 Minute Auflage werden die Probepapiere von der Tinte genommen, rückseitig auf Löschpapier abgequetscht und nach 5 Minuten beurteilt. Zur qualitativen Beurteilung der Durchdringung der Tinte durch das Papier und damit des Leimungsgrades wird eine Bewertung mit den Zahlen 1-5 durchgeführt, wobei die Zahlen im einzelnen bedeuten :

1 : Kein Tintendurchschlag
2 : 5-10 % Tintendurchschlag
3 : 10-20 % Tintendurchschlag

4 : ca. 50 % Tintendurchschlag
4,5* : ca. 90 % Tintendurchschlag
5 : 100 % Tintendurchschlag

Die folgende Tabelle gibt die Bewertung der Leimungswirkung des erfindungsgemäßen Leimungsmittels auf den Papieren A, B und C gemäß o. g. Schema wieder. Als Vergleich mit dem Stand der Technik wurde das als Wirksubstanz in vielen handelsüblichen Leimungsmitteln eingesetzte Stearoyldiketen verwendet.

Versuchsergebniss :

1. Papiersorte A : (kreidehaltig, neutral)

| Leimungsmittel | Feststoff i. Toluol | Bewertung der Leimung bei Nachbehandlung | | | | Durchschnitt der Bewertung |
|---|---|---|---|---|---|---|
| | | I | II | III | IV | |
| erfindungsgemäßes Leimungsmittel | 0,1 % | 1 | 3 | 2,5 | 2,5 | 2,25 |
| Stearoyldiketen | 0,1 % | 1 | 3,5 | 2,5 | 4,5 | 2,87 |

2. Papiersorte B : (kaolinhaltig, neutral)

| Leimungsmittel | Feststoff i. Toluol | Bewertung d. Leimung bei Nachbehandlung | | | | Durchschnitt der Bewertung |
|---|---|---|---|---|---|---|
| | | I | II | III | IV | |
| erfindungsgemäßes Leimungsmittel | 0,2 % | 3 | 4,5 | 4 | 4,5 | 4,0 |
| Stearoyldiketen | 0,2 % | 1,5 | 5 | 4,5 | 5 | 4,0 |

3. Papiersorte C : (kaolinhaltig, sauer)

| Leimungsmittel | Feststoff i. Toluol | Bewertung d. Leimung bei Nachbehandlung | | | | Durchschnitt der Bewertung |
|---|---|---|---|---|---|---|
| | | I | II | III | IV | |
| erfindungsgemäßes Leimungsmittel | 0,2 % | 2 | 3 | 4 | 4 | 3,25 |
| Stearoyldiketen | 0,2 % | 5 | 5 | 5 | 5 | 5 |

Die aufgeführten Versuchsergebnisse machen deutlich, daß das erfindungsgemäße Leimungsmittel auf Basis von disubstituiertem Monofluortriazin dem dem Stand der Technik entsprechenden Stearoyldiketen auf den Papiersorten A (kreidehaltig, neutral) und C (kaolinhaltig, sauer) überlegen und auf den Papiersorte B (kaolinhaltig, neutral) zumindest gleichwertig ist. Die Unterschiede in der Leimungswirkung dieser beiden Produkte kommen vor allem auf der Papiersorte C zum Ausdruck. Das in diesem Beispiel eingesetzte Papier, das Kaolin als Füllstoff enthält und unter Zusatz von Aluminiumsulfat in saurem Milieu hergestellt wurde, ist besonders praxisrelevant, da Papiere dieser Zusammensetzung häufig hergestellt werden.

Während dabei die mit Stearoyldiketen behandelten Papiere bei allen vier Arten der Nachbehandlung nach der Prüfzeit eine vollständige Tintendurchdringung zeigen, ist bei den mit dem erfindungsgemäßen Leimungsmitteln behandelten Prüflingen in derselben Prüfzeit nur eine wesentlich geringere

* Außerdem können noch weitere Zwischenwerte herangezogen werden.

Tintendurchdringung festzustellen, die bei 5-10 % (Nachbehandlung I), 10-20 % (Nachbehandlung II) und ca. 50 % (Nachbehandlung III und IV) liegt.

**Patentansprüche**

1. Monofluortriazin-Verbindungen der Formel

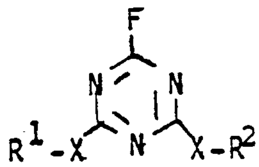

(1)

worin

$R^1$ und $R^2$ gleich oder verschieden sein können und für einen Kohlenwasserstoffrest mit 12 bis 24 C-Atomen stehen,

X für ein Sauerstoffatom, ein Schwefelatom oder die Gruppe $NR^3$ steht, worin

$R^3$ Wasserstoff oder einen Kohlenwasserstoffrest mit 1 bis 24 C-Atomen bedeutet.

2. Monofluortriazinyl-Verbindungen der Formel (1) gemäß Anspruch 1, worin

$R^1$ und $R^2$ gleich oder verschieden sein können und für lineares oder verzweigtes Alkyl ($C_{12}$-$C_{24}$) oder für Cycloalkyl ($C_{12}$-$C_{24}$) oder für aromatische Reste mit anellierten hydroaromatischen Systemen mit 12 bis 24 C-Atomen stehen und worin

$R^3$ für die vorgenannten Substituenten und für Methyl steht und worin

X die in Anspruch 1 angegebene Bedeutung hat.

3. Monofluortriazinyl-Verbindung der Formel

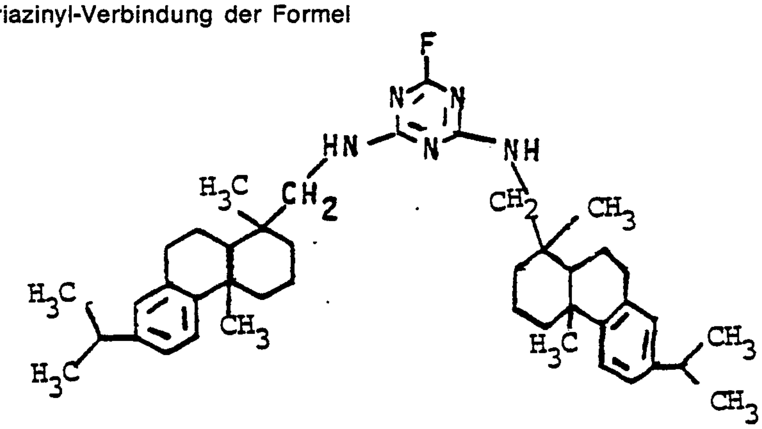

4. Monofluortriazinyl-Verbindung der Formel

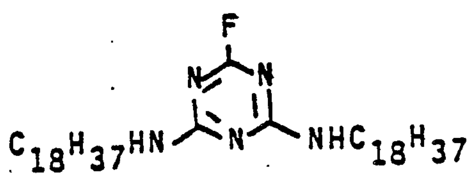

5. Monofluortriazinyl-Verbindung der Formel

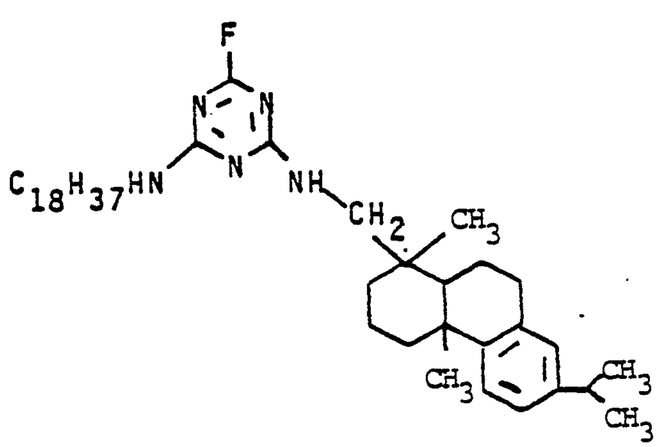

6. Verfahren zur Herstellung von Monofluortriazinyl-Verbindungen der Formel (1) gemäß Anspruch 1, dadurch gekennzeichnet, daß man Trifluortriazin mit Monoaminen, Monoalkoholen oder Mercaptanen der allgemeinen Formeln

$$R^1\text{—}XH \quad \text{und/oder} \quad R^2\text{—}XH$$

worin

$R^1$, $R^2$ und X die im Anspruch 1 angegebene Bedeutung besitzen, umsetzt.

7. Reaktivleimungsmittel für Papier, enthaltend als Wirksubstanz eine Monofluortriazinyl-Verbindung gemäß Anspruch 1 bis 5.

8. Reaktivleimungsmittel gemäß Anspruch 7, dadurch gekennzeichnet, daß das Leimungsmittel als Zusatz ein kationisches Fixiermittel enthält.

9. Verfahren zur Leimung von Papier mit Reaktivleimungsmitteln, dadurch gekennzeichnet, daß das Leimungsmittel als Wirksubstanz eine Verbindung gemäß Anspruch 1 bis 5 enthält.

10. Verfahren zur Leimung von Papier gemäß Anspruch 9, dadurch gekennzeichnet, daß das Leimungsmittel als Zusatz ein kationisches Fixiermittel enthält.

**Claims**

1. Monofluorotriazine compounds of the formula

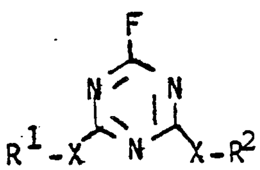

(1)

wherein

$R^1$ and $R^2$ can be identical or different and represent a hydrocarbon radical with 12 to 24 C atoms and X represents an oxygen atom, a sulphur atom or the group $NR^3$, wherein

$R^3$ denotes hydrogen or a hydrocarbon radical with 1 to 24 C atoms.

2. Monofluorotriazinyl compounds of the formula (1) according to Claim 1, wherein

$R^1$ and $R^2$ can be identical or different and represent linear or branched alkyl ($C_{12}$-$C_{24}$), cycloalkyl ($C_{12}$-$C_{24}$) or aromatic radicals with fused-on hydroaromatic systems with 12 to 24 C atoms, and wherein

$R^3$ represents the abovementioned substituents and methyl, and wherein

X has the meaning given in Claim 1.

3. Monofluorotriazinyl compound of the formula

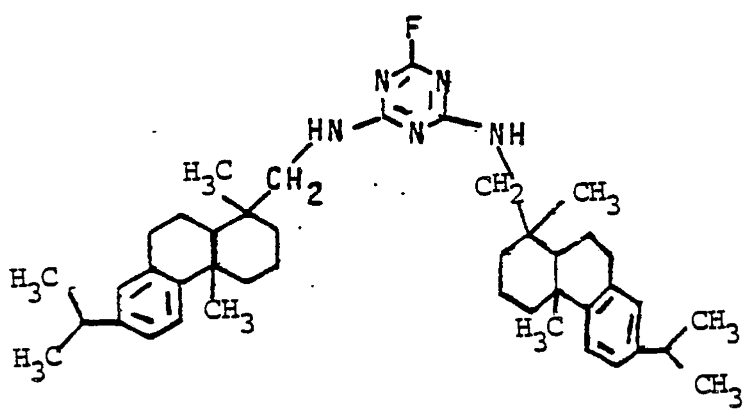

4. Monofluorotriazinyl compound of the formula

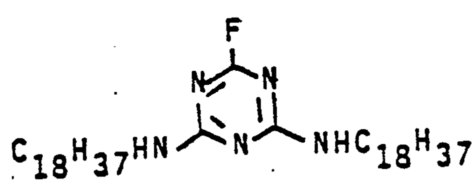

5. Monofluorotriazinyl compound of the formula

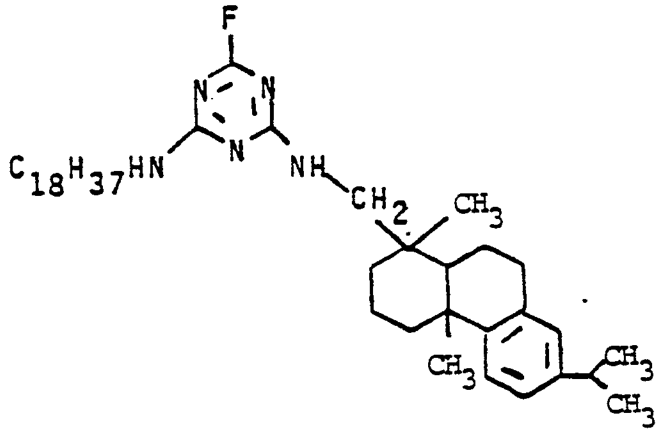

6. Process for the preparation of monofluorotriazinyl compounds of the formula (I) according to Claim 1, characterized in that trifluorotriazine is reacted with monoamines, monoalcohols or mercaptans of the general formulae

$$R^1—XH \quad \text{and/or} \quad R^2—XH$$

wherein

$R^1$, $R^2$ and X have the meaning given in Claim 1.

7. Reactive sizing agent for paper, containing, as the active substance, a monofluorotriazinyl compound according to Claim 1 to 5.

8. Reactive sizing agent according to Claim 7, characterized in that the sizing agent contains a cationic fixing agent as an additive.

9. Process for sizing paper with reactive sizing agent, characterised in that the sizing agent contains, as the active substance, a compound according to Claim 1 to 5.

10. Process for sizing paper according to Claim 9, characterised in that the sizing agent contains a cationic fixing agent as an additive.

**Revendications**

1. Composés de monofluorotriazine de formule

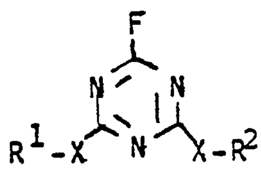

(1)

dans laquelle

$R^1$ et $R^2$ peuvent être identiques ou différents et représentent un reste d'hydrocarbure ayant 12 à 24 atomes de carbone,

X représente un atome d'oxygène, un atome de soufre ou le groupe $NR^3$, dans lequel

$R^3$ désigne l'hydrogène ou un reste d'hydrocarbure ayant 1 à 24 atomes de carbone.

2. Composés monofluorotriazinyliques de formule (1) suivant la revendication 1, dans lesquels

$R^1$ et $R^2$ peuvent être identiques ou différents et représentent un groupe alkyle (en $C_{12}$ à $C_{24}$) linéaire ou ramifié ou un groupe cycloalkyle (en $C_{12}$ à $C_{24}$) ou des restes aromatiques avec des systèmes hydroaromatiques condensés ayant 12 à 24 atomes de carbone et dans lesquels

$R^3$ représente les substituants mentionnés ci-dessus et le groupe méthyle et

X a la définition donnée dans la revendication 1.

3. Le composé monofluorotriazinylique de formule

(Voir formule page 10)

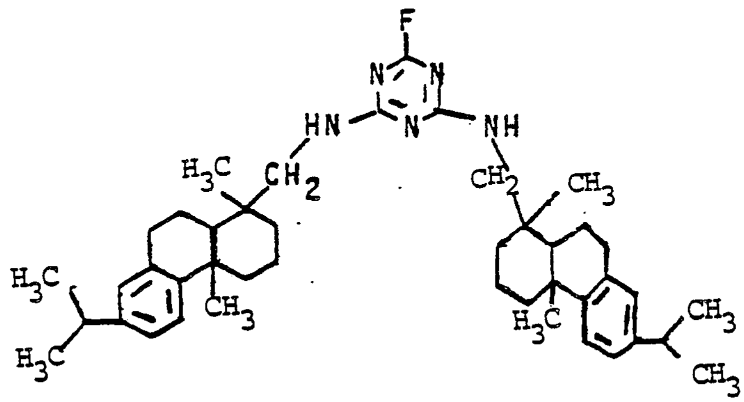

4. Le composé monofluorotriazinylique de formule

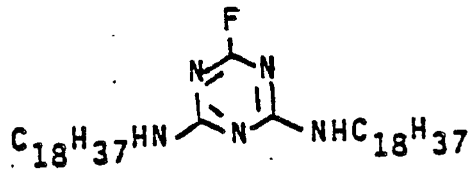

5. Le composé monofluorotriazinylique de formule

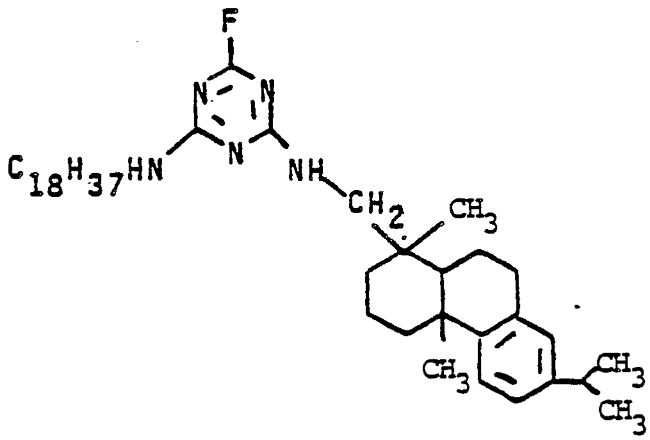

6. Procédé de production de composés monofluorotriazinylique de formule (1), suivant la revendication 1, caractérisé en ce qu'on fait réagir la trifluorotriazine avec des monoamines, des monoalcools ou des mercaptans de formules générales

$R^1$—XH et/ou $R^2$—XH

dans lesquelles

$R^1$, $R^2$ et X ont la définition indiquée dans la revendication 1.

7. Colles réactives pour papier, contenant comme substance active un composé monofluorotriazinylique suivant les revendications 1 à 5.

8. Colle réactive suivant la revendication 7, caractérisée en ce qu'elle contient comme additif un agent cationique de fixage.

9. Procédé pour le collage de papier avec des colles réactives, caractérisé en ce que la colle contient comme substance active un composé suivant les revendications 1 à 5.

10. Procédé de collage de papier suivant la revendication 9, caractérisé en ce que la colle contient comme additif un agent cationique de fixage.